(19) Europäisches Patentamt
European Patent Office
Office européen des brevets

(11) EP 4 807 363 A1

(12) EUROPEAN PATENT APPLICATION
published in accordance with Art. 153(4) EPC

(43) Date of publication:
**16.09.2026 Bulletin 2026/38**

(21) Application number: **24888673.1**

(22) Date of filing: **05.11.2024**

(51) International Patent Classification (IPC):
***G01N 33/53*** *(2006.01)* ***G01N 33/543*** *(2006.01)*

(52) Cooperative Patent Classification (CPC):
**G01N 33/53; G01N 33/543**

(86) International application number:
**PCT/JP2024/039183**

(87) International publication number:
**WO 2025/100378 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.11.2023 JP 2023189018**

(71) Applicant: **Fujirebio Inc.**
**Tokyo 107-0052 (JP)**

(72) Inventors:
- **NISHII, Tomonori**
  **Tokyo 107-0052 (JP)**
- **KATAGIRI, Noriko**
  **Hachioji-shi, Tokyo 192-0031 (JP)**
- **AOYAGI, Katsumi**
  **Tokyo 107-0052 (JP)**
- **KANEKO, Atsushi**
  **Tokyo 107-0052 (JP)**
- **NOJIMA, Hisashi**
  **Tokyo 107-0052 (JP)**
- **TADOKORO, Nanami**
  **Tokyo 107-0052 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) GPC3 MEASUREMENT METHOD AND KIT THEREFOR

(57) Disclosed are a method for measuring GPC3 and an immunoassay kit that reduce an increase in count values of negative samples. The method for measuring GPC3 is a method for measuring GPC3 by an immunoassay, comprising causing an antigen-antibody reaction between GPC3 contained in a liquid sample separated from a living body and a labeled anti-GPC3 antibody or an antigen-binding fragment thereof labeled with a labeling substance, and comprises contacting a solution containing the labeled anti-GPC3 antibody or an antigen-binding fragment thereof and a polyoxyethylene-polyoxypropylene block copolymer with GPC3 in the sample.

EP 4 807 363 A1

## Description

TECHNICAL FIELD

The present invention relates to a method of measuring GPC3 and a kit therefor.

BACKGROUND ART

Glypican-3 (GPC3) is a protein expressed during malignant transformation of hepatocytes and exists in a state immobilized on the cell membrane of hepatocytes. GPC3 is cleaved between the 358th arginine and the 359th serine by furin (furin cleavage site), and is divided into an N-terminal side subunit from the N-terminus to the 358th arginine and a C-terminal side subunit from the 359th serine to the C-terminus. The N-terminal side subunit and the C-terminal side subunit are linked by a single intramolecular disulfide bond.

Since GPC3 is also a protein for which specific expression is observed in cancers such as hepatocellular carcinoma, development of a method useful for testing cancer patients targeting GPC3 has been sought. For example, Patent Document 1 proposes a method of testing cancer patients by measuring GPC3. Also, Patent Document 2 proposes an immunoassay method of GPC3 using two different antibodies that bind to different epitopes present in the N-terminal region of GPC3. Patent Document 3 proposes a method of measuring GPC3 comprising subjecting a specimen containing GPC3 to a reduction treatment.

PRIOR ART DOCUMENTS

PATENT DOCUMENTS

Patent Document 1: WO 2004/038420
Patent Document 2: WO 2015/097928
Patent Document 3: WO 2022/154119

SUMMARY OF INVENTION

PROBLEMS TO BE SOLVED BY THE INVENTION

The present inventors have found that when measuring GPC3 in a liquid sample separated from a living body by an immunoassay, there is a problem that the count value of a liquid sample derived from a healthy individual (negative sample) increases. Therefore, an object of the present invention is to provide a method of measuring GPC3 and an immunoassay kit that reduced the increase in the count value of a negative sample.

MEANS FOR SOLVING THE PROBLEMS

As a result of intensive research, the present inventors have found that in a method of measuring GPC3 by an immunoassay, the increase in the count value of a negative sample can be reduced by adding a polyoxyethylene-polyoxypropylene block copolymer to a solution containing a labeled antibody, and completed the present invention.

That is, the present invention provides the following.

(1) A method of measuring GPC3 by an immunoassay, comprising a step of making GPC3 contained in a liquid sample separated from a living body and a labeled anti-GPC3 antibody or an antigen-binding fragment thereof labeled with a labeling substance undergo an antigen-antibody reaction, wherein the method comprises contacting a solution containing the labeled anti-GPC3 antibody or an antigen-binding fragment thereof and a polyoxyethylene-polyoxypropylene block copolymer with GPC3 in the sample.
(2) The method according to (1), wherein the immunoassay is a sandwich method.
(3) The method according to (1) or (2), wherein content of ethylene oxide in the polyoxyethylene-polyoxypropylene block copolymer is 70% or more and 90% or less.
(4) The method according to (3), wherein the number of moles of ethylene oxide added to the polyoxyethylene-polyoxypropylene block copolymer is 150 or more and 300 or less.
(5) The method according to any one of (1) to (4), wherein concentration of the polyoxyethylene-polyoxypropylene block copolymer in the solution containing the labeled anti-GPC3 antibody or an antigen-binding fragment thereof and

the polyoxyethylene-polyoxypropylene block copolymer is 0.01 w/v% to 6.0 w/v%.

(6) The method according to any one of (1) to (5), wherein the labeling substance is an enzyme.

(7) A kit for measuring GPC3, comprising a solution containing an anti-GPC3 antibody labeled with a labeling substance and a polyoxyethylene-polyoxypropylene block copolymer.

(8) Use of a solution containing an anti-GPC3 antibody labeled with a labeling substance and a polyoxyethylene-polyoxypropylene block copolymer for measuring GPC3.

(9) Use of a solution containing an anti-GPC3 antibody labeled with a labeling substance and a polyoxyethylene-polyoxypropylene block copolymer for the manufacture of a kit for measuring GPC3.

EFFECTS OF INVENTION

According to the present invention, it is possible to provide a method of measuring GPC3 and a measurement kit therefor in which an increase in the count value of a negative sample is suppressed.

MODE FOR CARRYING OUT INVENTION

The method of the present invention is a method of measuring GPC3 by mixing GPC3 contained in a liquid sample separated from a living body and a solution containing an anti-GPC3 antibody labeled with a labeling substance and a polyoxyethylene-polyoxypropylene block copolymer.

As shown in the Examples, it was found that when measuring GPC3 in a liquid sample separated from a living body by an immunoassay, the count value of a liquid sample derived from a healthy individual (negative sample) increases when a measurement reagent is stored for a prescribed period. By the fact that the solution containing the labeled anti-GPC3 antibody contains a polyoxyethylene-polyoxypropylene block copolymer, the increase in the count value of the negative sample can be suppressed.

In the present invention, GPC3 contained in a liquid sample separated from a living body is mainly soluble GPC3 secreted from GPC3-expressing cells. As GPC3, GPC3 derived from an arbitrary subject can be used. Such subjects are preferably mammals, and examples thereof include primates such as humans, monkeys, and chimpanzees; rodents such as mice and rats; lagomorphs such as rabbits; ungulates such as bovines, swines, goats, horses, and sheep; and carnivores such as dogs, cats, and ferrets. GPC3 is widely conserved in animals, and its amino acid sequence is highly conserved particularly among mammals. From the viewpoint of clinical application, the subject is preferably a human. Therefore, GPC3 is preferably human GPC3.

Human GPC3 is preferably an N-terminal fragment produced by cleavage between the 358th arginine residue and the 359th serine residue in human soluble GPC3 protein (Accession No.: P51654.1) consisting of 580 amino acid residues, or a soluble full-length GPC3 released by cleavage of the GPI anchor present in the C-terminus of human GPC3 protein. Examples of the soluble full-length GPC3 include a GPC3 fragment in which an N-terminal fragment and a C-terminal fragment produced by cleavage between the 358th arginine residue and the 359th serine residue in human GPC3 protein are linked to each other via a disulfide bond. More specifically, such human soluble GPC3 is (a) an N-terminal fragment consisting of amino acid residues at positions 1 to 358 in the amino acid sequence of SEQ ID NO: 1 or a variant thereof, (b) a soluble full-length GPC3 in which an N-terminal fragment consisting of amino acid residues at positions 1 to 358 in the amino acid sequence of SEQ ID NO: 1 or a variant thereof and a soluble C-terminal fragment consisting of amino acid residues at positions 359 to 560 in the amino acid sequence of SEQ ID NO: 1 or a variant thereof are linked by a disulfide bond, or (c) mutants thereof that can naturally occur between races and/or individuals. Such mutants are those into which one or more amino acid residue mutations (e.g., substitution, insertion, deletion) that can naturally occur between races and/or individuals have been introduced with respect to (a) the N-terminal fragment or a variant thereof, or (b) the soluble full-length GPC3 or a variant thereof. The number of amino acid residue mutations in such mutants may be, for example, 1 to 30, preferably 1 to 20, more preferably 1 to 15, even more preferably 1 to 10, particularly preferably 1, 2, 3, 4, or 5.

In the present invention, a liquid sample separated from a living body is an arbitrary liquid sample. Examples of the liquid sample include body fluids (e.g., blood, lymph, urine, milk, saliva, and tears) separated from a subject, extracts of tissues separated from a subject, washing fluids (e.g., those obtained by washing mucosal tissues such as the bronchial tubes) recovered from a subject, and liquids obtained from cell cultures derived from a subject, as well as liquid samples obtained by processing (e.g., fractionating) these. From the viewpoint of easy availability of a liquid sample rich in GPC3, the liquid sample is preferably a blood specimen (e.g., whole blood, serum, plasma).

In the present invention, a negative sample is a liquid sample derived from a subject who does not suffer from a target disease. A subject who does not suffer from a target disease is, for example, a subject who does not suffer from liver disease, preferably a subject who does not suffer from liver cancer, and more preferably a subject who does not suffer from hepatocellular carcinoma. A negative sample of GPC3 is, for example, a sample in which the concentration of GPC3 in the sample is 100.0 pg/mL or less, preferably 95.0 pg/mL or less. The concentration of GPC3 in the sample may be measured

for any of GPC3 in a state in which the N-terminal side subunit from the N-terminus to the 358th arginine, or the C-terminal side subunit from the 359th serine to the C-terminus, or the N-terminal side subunit and the C-terminal side subunit are connected.

In the present invention, an anti-GPC3 antibody refers to an antibody capable of specifically binding to GPC3. Examples of the antibody include polyclonal antibodies and monoclonal antibodies. From the viewpoint of obtaining an antibody having uniform reaction specificity with good reproducibility, the antibody is preferably a monoclonal antibody. Antibodies can also be specified by isotype. Examples of such isotypes include IgG, IgM, IgA, IgD, IgE, and IgY. Preferably, the antibody is IgG, IgM, or IgA, more preferably IgG or IgM, and even more preferably IgG. The antibody may further be a chimeric antibody, a humanized antibody, or a human antibody. The antibody may also be an antigen-binding fragment. The antigen-binding fragment may be any antibody fragment as long as it maintains the binding property (antigen-antibody reactivity) to the corresponding antigen of the original antibody, and may be a low-molecular-weight compound in which variable region fragments are linked. Specific examples include, but are not limited to, Fab, Fab', $F(ab')_2$, scFv, VHH antibody, and the like. In addition, "specifically bind" means having the ability to specifically bind, and means having the ability to undergo antigen-antibody reaction. The term "recognize" may be used with the same meaning as "specifically bind".

An anti-GPC3 monoclonal antibody or polyclonal antibody can be produced by a conventional method using GPC3 (preferably soluble GPC3 protein or a peptide thereof) as an immunogen. For example, in the case of a polyclonal antibody, the antibody of the present invention can be obtained by immunizing an animal with the above immunogen and generating the antibody from antiserum by a conventional method. In the case of a monoclonal antibody, the antibody of the present invention can be obtained by the following method. First, antibody-producing cells such as spleen cells of an animal immunized with the immunogen and tumor cells such as myeloma cells are fused with a fusing agent such as polyethylene glycol to prepare hybridomas. Next, hybridomas are selected using a selection medium such as IIAT medium, and monoclonalized and cultured by an appropriate method such as a limiting dilution method. Then, this culture supernatant is analyzed by an appropriate immunoassay method such as an enzyme immunoassay, and a clone producing the target anti-GPC3 antibody is selected, whereby the antibody of the present invention can be obtained. These methods for producing monoclonal antibodies can be performed by known methods, for example, the methods of Köhler and Milstein (Nature 256, 495-497 (1975)), Secher (Nature 285, 446-450 (1980)), and the like. Preparation of an antigen-binding fragment of an antibody is also known. For example, Fab and $F(ab')_2$ can be obtained by treating an antibody with a proteolytic enzyme such as papain or pepsin, as is well known. Methods for producing scFv (single chain fragment of variable region, single-chain antibody) and low-molecular-weight compounds are also well known, and they can be produced according to well-known methods. In addition, since anti-GPC3 antibodies and antigen-binding fragments thereof are commercially available, commercial products can also be used. Note that in the following description prior to the Examples, "antibody" means "antibody or an antigen-binding fragment thereof" unless it is clear otherwise from the context.

In the present invention, examples of the labeling substance include enzymes, fluorescent proteins, fluorescent substances, luminescent substances, radioactive substances, and dyes. The labeling substance is preferably an enzyme. Examples of the enzyme include alkaline phosphatase (ALP), peroxidase (POD (e.g., HRP)), luciferase, and β-galactosidase. Examples of the fluorescent protein include green fluorescent protein and red fluorescent protein. Examples of the fluorescent substance include fluorescein, fluorescein isothiocyanate, and rhodamine. Examples of the luminescent substance include acridinium derivatives. Examples of the radioactive substance include radioactive elements such as $^{3}H$, $^{14}C$, $^{32}P$, $^{35}S$, and $^{125}I$.

In the present invention, as a method of producing a labeled anti-GPC3 antibody, a conventionally known method or a method analogous thereto can be appropriately adopted, and the labeling substance and the anti-GPC3 antibody may be directly bonded or indirectly bonded.

Examples of a method of directly bonding include a method of adding an active group to a labeling substance or using a labeling substance having these active groups, and bonding an antibody by covalent bonding using the active group. When producing an enzyme-labeled anti-GPC3 antibody, for example, an enzyme-labeled anti-GPC3 antibody can be produced by mixing an enzyme into which an active ester group has been introduced and an anti-GPC3 antibody. In addition, an enzyme-labeled anti-GPC3 antibody can be produced by reacting an enzyme into which a maleimide group has been introduced with a reduced anti-GPC3 antibody.

As a method of indirectly immobilizing, for example, a labeling substance and an anti-GPC3 antibody can be indirectly bonded via a pair of affinity substances. Examples of the pair of affinity substances include combinations such as biotin and avidin (or streptavidin). For example, an antibody and a labeling substance can be indirectly bonded by bonding biotin to an anti-GPC3 antibody, bonding avidin (or streptavidin) to a labeling substance, and mixing the biotinylated antibody and the avidinated labeling substance.

In the present invention, the polyoxyethylene-polyoxypropylene block copolymer is a triblock copolymer of polyoxyethylene-polyoxypropylene-polyoxyethylene, and can be represented by $HO(C_2H_4O)_a$-$(C_3H_6O)_b$-$(C_2H_4O)_cH$ (Formula 1) (wherein a, b, and c represent arbitrary integers). Polyoxyethylene: $(C_2H_4O)_a$ or $(C_2H_4O)_c$ may be

represented by abbreviations such as EO and POE, and polyoxypropylene: $(C_3H_6O)_b$ may be represented by abbreviations such as PO, POP, and PPG, and similar notation may be used in this specification. The polyoxyethylene polyoxypropylene block copolymer is, for example, a poloxamer, and a commercially available one can be used. Note that there are several types of notation methods as components of the polyoxyethylene polyoxypropylene block copolymer, and identity and similarity can be confirmed between each notation, but even for commercial products with the same name, numerical values between each notation may not completely match. However, these are unique to polymerizable polymers and can be naturally understood by those skilled in the art.

For example, examples of the polyoxyethylene-polyoxypropylene block copolymer include poloxamer 388, poloxamer 407, poloxamer 188, poloxamer 217, poloxamer 237, poloxamer 238, poloxamer 288, poloxamer 108, and the like. Examples of commercial products of the polyoxyethylene-polyoxypropylene block copolymer include PLURONIC (registered trademark) series compounds such as Pluronic F108 (average molecular weight 14600, EO content about 80%, number of EO units (a+c) 264), Pluronic F127 (average molecular weight 12600, EO content about 70%, number of EO units (a+c) 202), Pluronic F68 (average molecular weight 8400, EO content about 80%, number of EO units (a+c) 160), Pluronic F77 (average molecular weight 6600, EO content about 70%, number of EO units (a+c) 104), Pluronic F87 (average molecular weight 7700, EO content about 70%, number of EO units (a+c) 128), Pluronic F88 (average molecular weight 11400, EO content about 80%, number of EO units (a+c) 207), Pluronic F98 (average molecular weight 13000, EO content about 80%, number of EO units (a+c) 236), and Pluronic F38 (average molecular weight 4700, EO content about 80%, number of EO units (a+c) 85).

The content of ethylene oxide (EO content, w/w%) in the polyoxyethylene-polyoxypropylene block copolymer is 60% or more and 95% or less, preferably 70% or more and 90% or less.

The number average molecular weight of the polyoxyethylene-polyoxypropylene block copolymer is, for example, 4,000 to 20,000, preferably 8,000 to 17,000, more preferably 9,000 to 15,000.

In addition, the average number of moles of ethylene oxide (number of EO units (a+c)) added to the polyoxyethylene-polyoxypropylene block copolymer is 100 or more and 300 or less, preferably 150 or more and 300 or less, more preferably 200 or more and 300 or less.

The polyoxyethylene-polyoxypropylene block copolymer is preferably poloxamer 388, poloxamer 407, or poloxamer 188, and more preferably poloxamer 388 or poloxamer 407.

In the present invention, the concentration of the polyoxyethylene-polyoxypropylene block copolymer in a solution containing the labeled anti-GPC3 antibody and the polyoxyethylene-polyoxypropylene block copolymer is not particularly limited as long as it is a concentration that can suppress an increase in the count value of a negative sample when measuring GPC3. Such a concentration is, for example, 0.001 to 10.0 w/v%, preferably 0.01 to 6.0 w/v%, more preferably 0.01 to 4.0 w/v%, even more preferably 0.25 to 4.0 w/v%.

In the present invention, the concentration of the labeled anti-GPC3 antibody in a solution containing the labeled anti-GPC3 antibody and the polyoxyethylene-polyoxypropylene block copolymer is not particularly limited as long as it is a concentration at which GPC3 can be measured. Such a concentration is, for example, 0.75 to 3.0 μg/mL, preferably 1.2 to 1.8 μg/mL.

In the present invention, the liquid serving as the base for the solution containing the labeled anti-GPC3 antibody and the polyoxyethylene-polyoxypropylene block copolymer is not particularly limited, and any aqueous liquid may be used. For example, water (e.g., distilled water, sterile water, sterile distilled water, pure water) and a buffer solution can be used. Examples of the buffer solution include phosphate buffer, MES buffer, citrate buffer, Tris buffer, carbonate buffer, HEPES buffer, and MOPS buffer. The pH of the buffer solution may be any pH at which the labeling substance and the anti-GPC3 antibody can be stably maintained, and may be 5.0 to 9.0 (preferably 6.0 to 8.0). The solution is preferably a buffer solution.

In the present invention, the solution containing the labeled anti-GPC3 antibody and the polyoxyethylene-polyoxypropylene block copolymer may further contain a water-soluble polymer such as BSA, a sugar such as sucrose, or a surfactant such as a nonionic surfactant, a cationic surfactant. an anionic surfactant, or a zwitterionic surfactant. As the surfactant, a nonionic surfactant is preferred.

The method of measuring GPC3 of the present invention is a method of measuring GPC3 by an immunoassay method using one or more types of antibodies against GPC3.

In the present invention, the immunoassay method is preferably any sandwich immunoassay method using two or more types of antibodies against GPC3, comprising a solution containing a labeled anti-GPC3 antibody (labeled antibody) and an anti-GPC3 antibody (solid-phase antibody) for capturing GPC3 on a solid phase. Sandwich immunoassay methods are well known in this field and can be carried out according to a well-known sandwich immunoassay method. Note that in the present invention, the term "measurement" includes detection, quantification, and semi-quantification.

In a sandwich immunoassay method (2-step sandwich immunoassay), for example, first, an anti-GPC3 antibody immobilized on a solid phase or an anti-GPC3 antibody that can be immobilized on a solid phase in a reaction step (solid-phase antibody) and GPC3 in a liquid sample are mixed, and the solid-phase antibody and GPC3 are made to undergo

antigen-antibody reaction to form an immune complex of GPC3 and the solid-phase antibody (primary reaction). When the solid-phase antibody is an anti-GPC3 antibody that can be immobilized on a solid phase, the solid-phase antibody may be immobilized on the solid phase after the primary reaction or simultaneously with the primary reaction. Thereafter, B/F separation is performed. Next, GPC3 bound to the solid-phase antibody and a solution containing a labeled anti-GPC3 antibody (labeled antibody) are mixed, and GPC3 and the labeled antibody are made to undergo antigen-antibody reaction to form an immune complex of the solid-phase antibody, GPC3, and the labeled antibody (secondary reaction). Next, B/F separation is performed, and GPC3 in the liquid sample can be measured by measuring a signal derived from the label of the labeled antibody bound to GPC3 captured on the solid phase (immune complex of the solid-phase antibody, GPC3, and the labeled antibody). Washing may be performed after B/F separation. In addition, the signal derived from the labeling substance of the immune complex of the solid-phase antibody, GPC3, and the labeled antibody may be measured in a state where the immune complex is formed, or after a part containing the labeling substance is dissociated from the immune complex, a signal derived from the dissociated labeling substance may be measured.

Also, first, GPC3 in a liquid sample and a solution containing a labeled antibody are mixed, and GPC3 and the labeled antibody are made to undergo antigen-antibody reaction to form an immune complex of GPC3 and the labeled antibody (primary reaction), after which a solid-phase antibody and GPC3 are made to undergo antigen-antibody reaction to form an immune complex of the solid-phase antibody, GPC3, and the labeled antibody (secondary reaction), and thereafter B/F separation may be performed. Next, GPC3 in the liquid sample can be measured by measuring a signal derived from the labeling substance of the labeled antibody bound to GPC3 bound to the solid phase. Washing may be performed after B/F separation.

Also, it is possible to simultaneously react the three of GPC3 in a liquid sample, a solid-phase antibody (anti-GPC3 antibody), and a labeled anti-GPC3 antibody (1-step sandwich immunoassay).

The conditions of the primary reaction and the secondary reaction are not particularly limited as long as the conditions are such that an antigen-antibody reaction is performed and an immune complex is formed, and reaction conditions can be appropriately set independently. For example, it can be performed at 4 to 45°C, preferably 20 to 37°C; at about pH 5.0 to 9.0, preferably pH 6.0 to 8.0, for about 1 minute to 12 hours, preferably about 3 minutes to 1 hour.

In the present invention, the immunoassay method can be classified into, for example, chemiluminescent enzyme immunoassay (CLEIA), chemiluminescent immunoassay (CLIA), enzyme immunoassay (EIA), radioimmunoassay (RIA), fluorescent immunoassay (FIA), etc., depending on the type of labeling substance used. Chemiluminescent enzyme immunoassay is an immunoassay using an enzyme as a label and using a substrate that generates a chemiluminescent compound as a substrate (for example, AMPPD etc. when alkaline phosphatase is used as an enzyme). Enzyme immunoassay is an immunoassay using an enzyme (for example, peroxidase, alkaline phosphatase, luciferase, β-galactosidase, etc.) as a label. As the substrate for each enzyme, a compound quantifiable by absorbance measurement or the like is used. For example, in the case of peroxidase, 1,2-phenylenediamine (OPD), 3,3',5,5'-tetramethylbenzidine (TMB), etc. are used; in the case of alkaline phosphatase, p-nitrophenyl phosphate (pNPP), etc. are used; in the case of β-galactosidase, MG: 4-methylumbelliferyl galactoside, NG: nitrophenyl galactoside, etc. are used; and in the case of luciferase, luciferin etc. are used. Radioimmunoassay (RIA) is a method using a radioactive substance as a label. Fluorescent immunoassay (FIA) is a method using a fluorescent substance or a fluorescent protein as a label. Immunoassays using these labels are *per se* well known in this field and are described, for example, in US 8039223 B and US 2015-0309016 Al.

In the immunoassay method described above, as a solid phase, for example, particles (e.g., Sepharose beads, agarose beads, magnetic particles), a support (e.g., membrane), and a container (e.g., plate such as plastic plate, tube, microfluidic channel) can be used, but from the viewpoint of automation and shortening of time, magnetic particles are preferred. Immobilization of an antibody to a solid phase may be performed by direct immobilization or indirect immobilization, and a conventional method can be used. For example, it can be immobilized by a physical adsorption method, a covalent bonding method, a method utilizing an affinity substance (e.g., biotin, streptavidin), and an ionic bonding method.

The present invention also provides a measurement kit for measuring GPC3 in a liquid sample by the measurement method of the present invention described above.

The measurement kit of the present invention comprises a solution containing the labeled anti-GPC3 antibody and the polyoxyethylene-polyoxypropylene block copolymer described above.

The measurement kit of the present invention may be a measurement kit by a sandwich immunoassay method, and may further comprise a solid-phase antibody. Preferred conditions for the labeling substance, anti-GPC3 antibody, polyoxyethylene-polyoxypropylene block copolymer, solution, measurement method, GPC3, etc. are the same as described above.

The measurement kit for GPC3 of the present invention may further comprise other reagents etc. generally included in measurement kits, such as an appropriate substrate solution and washing solution depending on the labeling substance to be used.

Hereinafter, the present invention will be specifically explained based on Examples. However, the present

invention is not limited to the following Examples. Note that in the following, the expression "%" indicates weight/volume percent (w/v%: g/100 mL) unless otherwise specified.

[Reference Example 1] Preparation of Anti-GPC3 Antibody A-Immobilized Particle Liquid

Anti-GPC3 antibody A recognizing the N-terminal side subunit of GPC3 was added to magnetic particles in 10 mM MES buffer (pH 5.0), and incubated at 25°C for 1 hour with gentle stirring. After the reaction, the magnetic particles were collected with a magnet, and the magnetic particles were washed with a washing solution to obtain anti-GPC3 antibody A-immobilized particles. At the time of measurement, the anti-GPC3 antibody A-immobilized particles were suspended in a particle dilution liquid (50 mM Tris buffer, 1mM EDTA 2Na, 0.1% $NaN_3$, 2.0% BSA, pH 7.2) to a final concentration of 0.05% to prepare an anti-GPC3 antibody A-immobilized particle liquid.

[Reference Example 2] Preparation of Alkaline Phosphatase-Labeled Anti-GPC3 Antibody

Anti-GPC3 antibody B recognizing the N-terminal side subunit of GPC3 was labeled with alkaline phosphatase (ALP) according to a conventional method, and a main peak was recovered and purified using column chromatography with Superdex 200 16/600 (trade name, manufactured by GE) with a buffer for purification (100 mM MES buffer, 150 mM NaCl, 0.1% $NaN_3$, pH 6.8) at a flow rate of 1.0 mL/min to obtain ALP-labeled anti-GPC3 antibody B.

ALP-labeled anti-GPC3 antibody C was prepared by the same method as above except that anti-GPC3 antibody C recognizing the C-terminal side subunit of GPC3 was used instead of anti-GPC3 antibody B. This was designated as ALP-labeled anti-GPC3 antibody C.

[Example 1]

<<Preparation of Solution Containing Alkaline Phosphatase-Labeled Anti-GPC3 Antibody>>

As a control, ALP-labeled anti-GPC3 antibody B was suspended in a labeled antibody dilution liquid containing no nonionic polymer (50 mM MES buffer, 150 mM NaCl, 0.3 mM $ZnCl_2$, 1 mM $MgCl_2$, 0.1% Proclin 300, 2.0% BSA, pH 6.8) to prepare an ALP-labeled anti-GPC3 antibody B liquid (Condition 1). Also, ALP-labeled anti-GPC3 antibody B was suspended in labeled antibody dilution liquids containing Pluronic F108, polyvinyl alcohol (PVA), and polyvinyl pyrrolidone (PVP), respectively, at a final concentration of 1.00% (final concentration of labeled antibody: 1.5 μg/mL) to prepare ALP-labeled anti-GPC3 antibody B liquids (Conditions 2 to 4).

ALP-labeled anti-GPC3 antibody C liquids were prepared by the same method as above except that ALP-labeled anti-GPC3 antibody C was used instead of ALP-labeled anti-GPC3 antibody B (Conditions 5 to 8).

<<Measurement of Negative Serum Specimens>>

As objects to be measured, two cases of negative serum specimens (hereinafter, negative serum specimens) which were specimens derived from healthy individuals (purchased from ProMedDX) were used. In both cases, the concentration of GPC3 is 95.0 pg/mL or less.

Negative serum specimens were evaluated with two types of measurement systems. One is a measurement system combining anti-GPC3 antibody A-immobilized particles and ALP-labeled anti-GPC3 antibody B (A-B measurement system), and the other is a measurement system combining anti-GPC3 antibody A-immobilized particles and ALP-labeled anti-GPC3 antibody C (A-C measurement system).

A method of measuring the A-B measurement system by a 2-step sandwich immunoassay (2STEP mode) is shown below. 50 μL of anti-GPC3 antibody A-immobilized particle liquid and 20 μL of a specimen were dispensed into a cuvette and mixed. Thereafter, incubation was performed at 37°C for 8 minutes, particles in the cuvette were collected with a magnet, and the inside of the cuvette was washed with a washing solution (0.05% Tween (registered trademark) 20/PBS). 50 μL of ALP-labeled anti-GPC3 antibody B liquid was dispensed into the cuvette, and after stirring, incubation was performed at 37°C for 8 minutes, particles in the cuvette were collected with a magnet, and the inside of the cuvette was washed with the washing solution. Thereafter, 200 μL of Lumipulse (registered trademark) substrate liquid (manufactured by Fujirebio Inc.) containing 3-(2'-spiroadamantane)-4-methoxy-4-(3"-phosphoryloxy)phenyl-1,2-dioxetane disodium salt (AMPPD), which is a chemiluminescent substrate, was dispensed into the cuvette, and after stirring and incubation at 37°C for 4 minutes, the amount of luminescence (counts) was measured with a luminometer. Measurement was performed with a fully automated chemiluminescent enzyme immunoassay system (Lumipulse L2400 (manufactured by Fujirebio Inc.)).

The method of measuring the A-C measurement system in the 2STEP mode was performed by the same method as above except that ALP-labeled anti-GPC3 antibody C liquid was used instead of ALP-labeled anti-GPC3 antibody B

liquid.

<<Calculation of Fluctuation Rate (%) of Count Value of Negative Serum Specimens by Maintaining Temperature >>

The anti-GPC3 antibody A-immobilized particle liquid was divided into two containers, and they were stored at 4°C and 37°C, respectively, for 3 days. Similarly, the ALP-labeled anti-GPC3 antibody B liquids (Conditions 1 to 4) under the four conditions described above and the ALP-labeled anti-GPC3 antibody C liquids (Conditions 5 to 8) under the four conditions described above were each divided into two containers, and they were stored at 4°C and 37°C, respectively, for 3 days. The anti-GPC3 antibody A-immobilized particle liquid stored at 4°C and the ALP-labeled anti-GPC3 antibody B liquids (Conditions 1 to 4) stored at 4°C were combined and measured in the 2STEP mode described above, and the anti-GPC3 antibody A-immobilized particle liquid stored at 37°C and the ALP-labeled anti-GPC3 antibody B liquids (Conditions 1 to 4) stored at 37°C were combined and measured in the 2STEP mode described above (A-B). The anti-GPC3 antibody A-immobilized particle liquid stored at 4°C and the ALP-labeled anti-GPC3 antibody C liquids (Conditions 5 to 8) stored at 4°C were combined and measured in the 2STEP mode, and the anti-GPC3 antibody A-immobilized particle liquid stored at 37°C and the ALP-labeled anti-GPC3 antibody C liquids (Conditions 5 to 8) stored at 37°C were combined and measured in the 2STEP mode (A-C).

The fluctuation rate (%) of the count value of a negative serum specimen was calculated using (count value at 37°C) / (count value at 4°C) $\times$ 100 (Formula 2). (Formula 2).

Count values measured by the A-B measurement system and fluctuation rates (%) of the count values of negative serum specimens are shown in Table 1, and count values measured by the A-C measurement system and fluctuation rates (%) of the count values of negative serum specimens are shown in Table 2.

[Table 1]

| A-B Measurement System | | | | | | | | | |
|---|---|---|---|---|---|---|---|---|---|
| Condition | | Condition 1 | | Condition 2 | | Condition 3 | | Condition 4 | |
| Type of polymer in labeled body dilution liquid | | - | | Pluronic F108 | | PVA | | PVP | |
| Concentration of polymer in labeled body dilution liquid | | - | | 1.00% | | 1.00% | | 1.00% | |
| Storage temperature | | 4°C | 37°C | 4°C | 37°C | 4°C | 37°C | 4°C | 37°C |
| Count value | Negative serum specimen A | 7,808 | 31,420 | 5,291 | 7,210 | 7,442 | 23.643 | 7,380 | 25,722 |
| | Negative serum specimen B | 5,208 | 29.923 | 3,679 | 5,399 | 5.634 | 20.969 | 5,268 | 21.147 |
| Fluctuation rate | Negative serum specimen A | | 402% | | 136% | | 318% | | 349% |
| | Negative serum specimen B | | 575% | | 147% | | 372% | | 401% |

[Table 2]

| A-C Measurement System | | | | |
|---|---|---|---|---|
| Condition | Condition 5 | Condition 6 | Condition 7 | Condition 8 |
| Type of polymer in labeled body dilution liquid | - | Pluronic F108 | PVA | PVP |

(continued)

| A-C Measurement System | | | | | | | | | |
|---|---|---|---|---|---|---|---|---|---|
| Condition | | Condition 5 | | Condition 6 | | Condition 7 | | Condition 8 | |
| Concentration of polymer in labeled body dilution liquid | | - | | 1.00% | | 1.00% | | 1.00% | |
| Storage temperature | | 4°C | 37°C | 4°C | 37°C | 4°C | 37°C | 4°C | 37°C |
| Count value | Negative serum specimen A | 2,129 | 3,017 | 1,865 | 2,051 | 2.349 | 3.123 | 2,106 | 3,478 |
| | Negative serum specimen B | 1,912 | 3,025 | 1,762 | 1,915 | 2,090 | 2,940 | 1,944 | 2,916 |
| Fluctuation rate | Negative serum specimen A | | 142% | | 110% | | 133% | | 165% |
| | Negative serum specimen B | | 158% | | 109% | | 141% | | 150% |

The fluctuation rate (%) of the count value of a negative serum specimen is an index for evaluating the influence on measurement when a measurement reagent is maintained at an elevated temperature for a prescribed period. It can be said that the closer the fluctuation rate is to 100%, the more robust the reagent is, which is not affected by temperature.

Table 1 shows results measured by the A-B measurement system. When a labeled body dilution liquid containing no nonionic polymer was used, the count value of the negative serum specimen significantly increased when stored at 37°C as compared with when stored at 4°C (Table 1, Condition 1). The fluctuation rate of the count value of the negative serum specimen was 400% or more. This indicates that when measuring a negative serum specimen containing a small amount of GPC3, it is affected by temperature during storage of the measurement reagent. On the other hand, in the condition using a labeled body dilution liquid containing Pluronic F108 (Table 1, Condition 2), the fluctuation rate of the negative serum specimen was about 140%, and it was found that the influence of temperature can be reduced. Also, in conditions using labeled body dilution liquids containing PVA or PVP, which are other nonionic polymers (Table 1, Conditions 3 and 4), the fluctuation rate was about 350 to 400%, and it was found that the condition using the labeled body dilution liquid containing Pluronic F108 can most reduce the influence of temperature. Similarly, when a positive sample containing 100.0 pg/mL or more of GPC3 was measured under the same conditions as Condition 1, the fluctuation rate of the count value of the positive sample was within 100 to 110% (data not shown).

Table 2 shows results measured by the A-C measurement system. Even when measured by the A-C measurement system, the results showed effects similar to those obtained by the A-B measurement system.

From the above, it was suggested that regardless of the type of anti-GPC3 antibody, when a labeled body dilution liquid containing Pluronic F108 is used, a highly robust reagent that is not affected by temperature is obtained.

[Example 2]

<<Preparation of Solution Containing Alkaline Phosphatase-Labeled Anti-GPC3 Antibody>>

As a control, ALP-labeled anti-GPC3 antibody B was suspended in a labeled antibody dilution liquid containing no nonionic polymer (final concentration of labeled antibody: 1.5 μg/mL) to prepare an ALP-labeled anti-GPC3 antibody B liquid (Condition 9).

In order to examine effective concentrations in the case of containing Pluronic F108, ALP-labeled anti-GPC3 antibody B was suspended in labeled antibody dilution liquids containing Pluronic F108 at final concentrations of 0.01, 0.05, 0.1, 0.25, 0.5, 1.0, 2.0, 4.0%, and 6.0% (final concentration of labeled antibody: 1.5 μg/mL) (Conditions 10 to 18). In order to examine other Pluronic-based polymers, ALP-labeled anti-GPC3 antibody B was suspended in labeled antibody dilution liquids containing Pluronic F68 at final concentrations of 0.05, 0.25, and 1.00% (final concentration of labeled antibody: 1.5 μg/mL) (Conditions 19 to 21), and ALP-labeled anti-GPC3 antibody B was suspended in labeled antibody dilution liquids containing Pluronic F127 at final concentrations of 0.05, 0.25, and 1.00% (final concentration of labeled antibody: 1.5 μg/mL) (Conditions 22 to 24) to prepare ALP-labeled anti-GPC3 antibody B liquids.

<<Measurement of Negative Serum Specimens (2STEP)>>

Two cases of negative serum specimens similar to those in Example 1 were used as objects to be measured.

<<Calculation of Fluctuation Rate (%) of Count Value of Negative Serum Specimens by Maintaining Temperature>>

An anti-GPC3 antibody A-immobilized particle liquid prepared by a method similar to that described in Example 1 was divided into three containers, and they were stored at 4°C, 25°C, and 37°C, respectively, for 3 days. The ALP-labeled anti-GPC3 antibody B liquid under Condition 11 described above was divided into three containers, and they were stored at 4°C, 25°C, and 37°C, respectively, for 3 days. The ALP-labeled anti-GPC3 antibody B liquids under Conditions 12 to 26 with different labeled antibody dilution liquids described above were each divided into two containers, and they were stored at 4°C and 37°C, respectively, for 3 days. The anti-GPC3 antibody A-immobilized particle liquids stored at 4°C, 25°C, and 37°C and the ALP-labeled anti-GPC3 antibody B liquids stored at 4°C, 25°C, and 37°C were combined at the same temperatures and measured in the 2STEP mode (Condition 9). The anti-GPC3 antibody A-immobilized particle liquids stored at 4°C and 37°C and the ALP-labeled anti-GPC3 antibody B liquids stored at 4°C and 37°C were combined at the same temperatures and measured in the 2STEP mode (Conditions 10 to 24).

Count values and fluctuation rates (%) of count values of negative serum specimens were calculated in the same manner as in Example 1. Results are shown in Table 3.

[Table 3-1]

| Condition | | Condition 9 | | | Condition 10 | | Condition 11 | | Condition 12 | | Condition 13 | | Condition 14 | | Condition 15 | | Condition 16 | |
|---|---|---|---|---|---|---|---|---|---|---|---|---|---|---|---|---|---|---|
| Type of polymer in labeled body dilution liquid | | - | | | Pluronic F108 | | | | | | | | | | | | | |
| Concentration of polymer in labeled body dilution liquid | | - | | | 0.01% | | 0.05% | | 0.10% | | 0.25% | | 0.50% | | 1.00% | | 2.00% | |
| Storage temperature | | 4°C | 25°C | 37°C | 4°C | 37°C | 4°C | 37°C | 4°C | 37°C | 4°C | 37°C | 4°C | 37°C | 4°C | 37°C | 4°C | 37°C |
| Count value | Negative serum specimen A | 7,573 | 8,609 | 28,846 | 6,558 | 22,784 | 5,971 | 13,441 | 6,071 | 9,817 | 5,221 | 9,020 | 5,298 | 7,741 | 5,409 | 7,231 | 5,908 | 7,893 |
| | Negative serum specimen B | 5,535 | 6,272 | 26,938 | 4,959 | 22,691 | 4,531 | 14,068 | 3,938 | 10,207 | 3,751 | 6,948 | 3,680 | 5,689 | 3,705 | 5,201 | 4,134 | 5,721 |
| Fluctuation rate | Negative serum specimen A | | 114% | 381% | | 347% | | 225% | | 162% | | 173% | | 146% | | 134% | | 134% |
| | Negative serum specimen B | | 113% | 487% | | 458% | | 310% | | 259% | | 185% | | 155% | | 140% | | 138% |

[Table 3-2]

| Condition | | Condition 17 | | Condition 18 | | Condition 19 | | Condition 20 | | Condition 21 | | Condition 22 | | Condition 23 | | Condition 24 | |
|---|---|---|---|---|---|---|---|---|---|---|---|---|---|---|---|---|---|
| Type of polymer in labeled body dilution liquid | | Pluronic F108 | | | | Pluronic F68 | | | | | | Pluronic F127 | | | | | |
| Concentration of polymer in labeled body dilution liquid | | 4.00% | | 6.00% | | 0.05% | | 0.25% | | 1.00% | | 0.05% | | 0.25% | | 1.00% | |
| Storage temperature | | 4°C | 37°C | 4°C | 37°C | 4°C | 37°C | 4°C | 37°C | 4°C | 37°C | 4°C | 37°C | 4°C | 37°C | 4°C | 37°C |
| Count value | Negative serum specimen A | 6,796 | 9,109 | 9,408 | 27,228 | 7,153 | 27,422 | 6,422 | 18,570 | 5,627 | 14,989 | 5,713 | 13,858 | 5,385 | 9,127 | 5,695 | 7,563 |
| | Negative serum specimen B | 4,655 | 6,551 | 6,547 | 25,968 | 4,951 | 24,307 | 4,263 | 15,688 | 3,973 | 11,994 | 4,006 | 10,616 | 3,761 | 6,454 | 3,685 | 5,491 |
| Fluctuation rate | Negative serum specimen A | | 134% | | 289% | | 383% | | 289% | | 266% | | 243% | | 169% | | 133% |
| | Negative serum specimen B | | 141% | | 397% | | 491% | | 368% | | 302% | | 265% | | 172% | | 149% |

When labeled antibody dilution liquids containing Pluronic F108 at 0.01% to 6.0% were used, the fluctuation rate of negative serum specimens was lower than when a labeled antibody dilution liquid containing no nonionic polymer was used, and it was found that there is an effect of reducing the influence of temperature (Table 3, Conditions 10 to 18). Furthermore, when labeled antibody dilution liquids containing Pluronic F108 at 0.25% to 4.0% were used, the fluctuation rate of negative serum specimens was lower than 200%, and it was found that the effect of reducing the influence of temperature is higher (Table 3, Conditions 13 to 17).

When labeled antibody dilution liquids containing Pluronic F68 at 0.25% to 1.00% were used, the fluctuation rate of negative serum specimens was lower than when a labeled antibody dilution liquid containing no nonionic polymer was used, and it was found that there is an effect of reducing the influence of temperature (Table 3, Conditions 19 to 21).

When labeled antibody dilution liquids containing Pluronic F127 at 0.05% to 1.00% were used, the fluctuation rate of negative serum specimens was lower than when a labeled antibody dilution liquid containing no nonionic polymer was used, and it was found that there is an effect of reducing the influence of temperature (Table 3, Conditions 22 to 24). Conditions including labeled antibody dilution liquids containing Pluronic F127 showed effects equivalent to those of conditions including labeled antibody dilution liquids containing Pluronic F108.

## Claims

1. A method of measuring GPC3 by an immunoassay, comprising a step of making GPC3 contained in a liquid sample separated from a living body and a labeled anti-GPC3 antibody or an antigen-binding fragment thereof labeled with a labeling substance undergo an antigen-antibody reaction, wherein the method comprises contacting a solution containing the labeled anti-GPC3 antibody or an antigen-binding fragment thereof and a polyoxyethylene-polyoxypropylene block copolymer with GPC3 in the sample.

2. The method according to claim 1, wherein the immunoassay is a sandwich method.

3. The method according to claim 1 or 2, wherein content of ethylene oxide in the polyoxyethylene-polyoxypropylene block copolymer is 70% or more and 90% or less.

4. The method according to claim 3, wherein the number of moles of ethylene oxide added to the polyoxyethylene-polyoxypropylene block copolymer is 150 or more and 300 or less.

5. The method according to claim 1 or 2, wherein concentration of the polyoxyethylene-polyoxypropylene block copolymer in the solution containing the labeled anti-GPC3 antibody or an antigen-binding fragment thereof and the polyoxyethylene-polyoxypropylene block copolymer is 0.01 w/v% to 6.0 w/v%.

6. The method according to claim 1 or 2, wherein the labeling substance is an enzyme.

7. A kit for measuring GPC3, comprising a solution containing an anti-GPC3 antibody labeled with a labeling substance and a polyoxyethylene-polyoxypropylene block copolymer.

8. Use of a solution containing an anti-GPC3 antibody labeled with a labeling substance and a polyoxyethylene-polyoxypropylene block copolymer for measuring GPC3.

9. Use of a solution containing an anti-GPC3 antibody labeled with a labeling substance and a polyoxyethylene-polyoxypropylene block copolymer for the manufacture of a kit for measuring GPC3.

## INTERNATIONAL SEARCH REPORT

International application No.
**PCT/JP2024/039183**

**A. CLASSIFICATION OF SUBJECT MATTER**

***G01N 33/53***(2006.01)i; ***G01N 33/543***(2006.01)i
FI: G01N33/53 D; G01N33/543 545J

According to International Patent Classification (IPC) or to both national classification and IPC

**B. FIELDS SEARCHED**

Minimum documentation searched (classification system followed by classification symbols)

G01N33/53; G01N33/543

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); Scopus

**C. DOCUMENTS CONSIDERED TO BE RELEVANT**

| Category* | Citation of document, with indication, where appropriate, of the relevant passages | Relevant to claim No. |
|---|---|---|
| Y | WO 2022/154119 A1 (FUJIREBIO INC.) 21 July 2022 (2022-07-21)<br>paragraphs [0010]-[0011], [0013], [0015]-[0017], [0019], [0024], [0034]-[0035], [0044] | 1-9 |
| Y | HAWI, Sharon R. et al. Detection of Membrane-Bound Enzymes in Cells Using Immunoassay and Raman Microspectroscopy. Analytical Biochemistry. 1998, vol. 259, no. 2, pp. 212-217, doi.org/10.1006/abio.1998.2661<br>p. 213, right column, 3rd paragraph | 1-9 |
| Y | JP 2015-7652 A (KYOWA MEDEX CO., LTD.) 15 January 2015 (2015-01-15)<br>claims 1-2, paragraphs [0021]-[0022] | 1-9 |
| Y | WO 2010/065797 A2 (KOHNE, David, E.) 10 June 2010 (2010-06-10)<br>paragraphs [0004]-[0005], [0012], [0019], [0022], [0033]-[0036], [0105]-[0106] | 1-9 |
| A | WO 2016/017037 A1 (BEACLE INC.) 04 February 2016 (2016-02-04)<br>entire text, all drawings | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 November 2024** | **10 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/039183**

**C. DOCUMENTS CONSIDERED TO BE RELEVANT**

| Category* | Citation of document, with indication, where appropriate, of the relevant passages | Relevant to claim No. |
|---|---|---|
| A | JP 2014-507160 A (CARIS LIFE SCIENCES LUXEMBOURG HOLDINGS S A R L) 27 March 2014 (2014-03-27) entire text, all drawings | 1-9 |
| A | NOJIMA, Hisashi et al. Elucidation of the Usefulness of Glypican-3, a Hepatocellular Carcinoma Biomarker, with the Fully Automated LUMIPULSE System. BPB Reports. 15 February 2023, vol. 6, no. 1, pp. 21-26, doi: 10.1248/bpbreports.6.1_21 entire text, all drawings | 1-9 |

Form PCT/ISA/210 (second sheet) (July 2022)

**INTERNATIONAL SEARCH REPORT**

International application No.
**PCT/JP2024/039183**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/039183**

| Patent document cited in search report | Publication date (day/month/year) | Patent family member(s) | Publication date (day/month/year) |
|---|---|---|---|
| WO 2022/154119 A1 | 21 July 2022 | US 2024/0094210 A1<br>paragraphs [0015]-[0016], [0018], [0020]-[0022], [0024], [0029], [0041]-[0044], [0054]<br>EP 4279504 A1<br>CN 116802495 A | |
| JP 2015-7652 A | 15 January 2015 | US 2012/0219966 A1<br>claims 1-2, paragraphs [0022]-[0023]<br>WO 2011/052620 A1<br>EP 2495565 A1<br>EP 2919011 A1<br>CN 102687015 A<br>KR 10-2012-0101421 A<br>CN 104034882 A<br>KR 10-2017-0075017 A<br>CN 106959367 A | |
| WO 2010/065797 A2 | 10 June 2010 | (Family: none) | |
| WO 2016/017037 A1 | 04 February 2016 | US 2016/0202251 A1<br>entire text, all drawings<br>CN 105492605 A | |
| JP 2014-507160 A | 27 March 2014 | US 2014/0141986 A1<br>entire text, all drawings<br>US 2014/0220580 A1<br>WO 2013/022995 A2<br>CN 103797131 A<br>KR 10-2014-0072014 A<br>CN 103874770 A<br>KR 10-2014-0067001 A<br>JP 2014-519340 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004038420 A
- WO 2015097928 A
- WO 2022154119 A
- US 8039223 B
- US 20150309016 A1


### Non-patent literature cited in the description


- **KÖHLER** ; **MILSTEIN**. *Nature*, 1975, vol. 256, 495-497
- **SECHER**. *Nature*, 1980, vol. 285, 446-450